# EUROPEAN PATENT APPLICATION

(11) **EP 4 744 619 A1**
(43) Date of publication of application: **20.05.2026**
(21) Application number: 24921152.5
(22) Date of filing: 21.03.2024
(51) Int. Cl.: A61F 2/04

(54) **AUTOMATICALLY BREAKING DIGESTIVE TRACT SLEEVE AND KIT**

(30) Priority: 29.01.2024 CN 202410125123
(71) Applicant: Hangzhou Tangji Medical Technology Co., Ltd., Hangzhou, Zhejiang 310052 (CN)
(72) Inventor: LI, Wenyu, Hangzhou, Zhejiang 310052 (CN)
(74) Representative: Vogelbruch, Keang
(86) International application number: PCT/CN2024/082937
(87) International publication number: WO 2025/161106

(57) **Abstract**

Provided are an automatically breaking digestive tract sleeve (100) and a kit. The automatically breaking digestive tract sleeve (100) has a length of 60-150 cm. The sleeve (100) comprises a proximal end (101) and a distal end (102). At least one degradable tube section (110) made of a degradable material is arranged on the sleeve (100) at a position 30-120 cm from the proximal end (101). The digestive tract implantation kit comprises a support (200) and the sleeve (100). The provided sleeve (100) effectively prevents intestinal obstruction caused by device blockage, thereby significantly improving the safety of in vivo device implantation.

## Description

### CROSS-REFERENCE TO RELATED APPLICATIONS

The present disclosure claims priority to Chinese Patent Application No. 202410125123.1, filed with the China National Intellectual Property Administration on January 29, 2024 and entitled "AUTOMATICALLY BREAKING DIGESTIVE TRACT SLEEVE AND KIT", which is incorporated herein by reference in its entirety.

### TECHNICAL FIELD

The present disclosure relates to the technical field of medical devices, and specifically, to a self-fragmenting (automatically breaking) digestive tract sleeve and an assembly (kit).

### BACKGROUND

Currently, obesity affects a large number of people worldwide, and scientific evidence shows that excessive obesity can lead to a series of diseases, such as diabetes. According to data released by the International Diabetes Federation in December 2021, there are currently 537 million adults aged 20-79 with diabetes, accounting for 10.5% of the global population in this age group.

Since 2008, GI Dynamics Inc., a U.S.-based company, has developed a duodenal-jejunal bypass liner (DJBL) inspired by the principles of Roux-en-Y gastric bypass (RYGB), a type of metabolic surgery. According to the DJBL, a sleeve is placed in the proximal duodenum and jejunum, isolating chyme from contact with the intestinal wall. Nutrients ingested pass from the stomach into the proximal jejunum through the sleeve; and pancreatic juice and bile are naturally secreted and flow downward between the sleeve and the intestinal wall, mixing with the chyme at the distal end of the DJBL (i.e., the jejunum). This design reduces nutrient absorption and aims to treat obesity and type 2 diabetes. However, studies have shown that after implantation, the sleeve has a certain probability (approximately 5%) of causing device obstruction. When device obstruction occurs, mild cases require early removal of the device, while severe cases can lead to intestinal obstruction or even intussusception. Therefore, improving the safety of the sleeve after implantation is crucial.

In view of this, the present disclosure is hereby proposed.

### SUMMARY

An objective of the present disclosure is to provide a self-fracturing digestive tract sleeve and an assembly, which is intended to improve at least one of the problems in the prior art.

The present disclosure is implemented as follows.

In a first aspect, the present disclosure provides a self-fracturing digestive tract sleeve, where the sleeve has a length of 60-150cm and includes a proximal end and a distal end, and at least one degradable sleeve segment (tube section) made of a degradable material is provided on the sleeve at a position 30-120cm from the proximal end.

In an optional embodiment, the degradable material includes at least one of poly(DL-lactide-co-glycolide), poly(ethylene glycol)-poly(ε-caprolactone), poly(L-lactide-co-ε-caprolactone), poly(L-lactic acid), poly(L-lactic acid)-trimethyl chitosan, and poly(p-dioxanone).

In an optional embodiment, after the sleeve is implanted into the digestive tract, an expected degradation time of the degradable sleeve segment is 30-270 days.

In an optional embodiment, a plurality of degradable sleeve segments are provided, and the plurality of degradable sleeve segments are uniformly distributed along a length direction of the sleeve.

In an optional embodiment, a preset degradation time of the plurality of degradable sleeve segments decreases sequentially from the proximal end to the distal end.

In an optional embodiment, other portions of the sleeve, excluding the degradable sleeve segments, are made of at least one material selected from PTFE, FEP, ePTFE, PU, LDPE, LLDPE, and PE.

In an optional embodiment, the sleeve has a wall thickness of 0.01-0.05 mm.

In an optional embodiment, the degradable sleeve segments are connected to the other portions of the sleeve by calendering or welding.

In an optional embodiment, the welding is laser welding or RF welding.

In a second aspect, the present disclosure provides a digestive tract implant assembly, including a stent (support) and the self-fracturing digestive tract sleeve according to any of the foregoing embodiments, where the proximal end of the self-fracturing digestive tract sleeve is connected to the stent.

The present disclosure has the following beneficial effects.

The self-fracturing digestive tract sleeve obtained through the above design includes at least one degradable sleeve segment. This design ensures that after implantation of the sleeve in the digestive tract for a period of time, the degradable sleeve degrades and fractures in the digestive tract environment, thereby causing the sleeve segment from the degradation site to the distal end to detach. By presetting the degradation time to occur before potential device obstruction, the detachment of the sleeve segment closer to the distal end can effectively prevent intestinal obstruction caused by device obstruction. Alternatively, even if the degradable sleeve segment has not fully degraded before device obstruction occurs, the mechanical performance of the degradable sleeve segment is compromised due to partial degradation. Under intestinal peristaltic forces, it can still fracture and detach when device obstruction occurs. In this connection, the sleeve provided by the embodiment of the present disclosure effectively prevents intestinal obstruction caused by device obstruction, thereby significantly improving the safety of device after implantation in the body.

### BRIEF DESCRIPTION OF THE DRAWINGS

To more clearly illustrate the technical solutions of the embodiments of the present disclosure, the drawings required in the embodiments of the present disclosure will be briefly described below. It should be understood that the following drawings only illustrate some embodiments of the present disclosure and therefore should not be considered as limitations of the scope. For those of ordinary skill in the art, other related drawings can be obtained according to these drawings without creative efforts.
FIG. 1 is a schematic structural diagram of a structure of a sleeve in cooperation with a stent according to an embodiment of the present disclosure; and
FIG. 2 is a schematic diagram illustrating the occurrence of simulating intestinal obstruction in an experimental example.

Reference numerals: 100. sleeve; 101. proximal end; 102. distal end; 110. degradable sleeve segment; 200. stent; 300. simulator; 301. adjustable valve; and 302. placement port.

### DETAILED DESCRIPTION OF THE EMBODIMENTS

To make the objectives, technical solutions, and advantages of embodiments of the present disclosure clearer, the following clearly and completely describes the technical solutions in embodiments of the present disclosure. Unless otherwise specified, the experimental conditions described in the examples are conducted under conventional conditions or in accordance with conditions recommended by manufacturers. Unless otherwise indicated, reagents and instruments used in the examples are conventional products that can be purchased commercially.

The features and performance of the present disclosure are further described in detail below in combination with the embodiments.

The self-fracturing digestive tract sleeve 100 and its assembly provided by the present disclosure are described in detail below.

The self-fracturing digestive tract sleeve 100 provided by the embodiment of the present disclosure has a length of 60-150 cm. The sleeve 100 includes a proximal end 101 and a distal end 102. At least one degradable sleeve segment 110 made of a degradable material is provided on the sleeve 100 at a position spaced from the proximal end 101.

The sleeve 100 provided by the embodiment of the present disclosure includes at least one degradable sleeve segment 110. This design ensures that after implantation of the sleeve 100 in the digestive tract for a period of time, the degradable sleeve 100 degrades and fractures in the digestive tract environment, thereby causing the sleeve segment from the degradation site to the distal end 102 to detach. By presetting the degradation time to occur before potential device obstruction, the detachment of the sleeve segment closer to the distal end 102 can effectively prevent intestinal obstruction caused by device obstruction. Alternatively, even if the degradable sleeve segment 110 has not fully degraded before device obstruction occurs, the mechanical performance of the degradable sleeve segment 110 is compromised due to partial degradation. Consequently, under peristaltic forces, it can still fracture and detach when device obstruction occurs.

Therefore, the sleeve 100 provided by the embodiment of the present disclosure effectively prevents intestinal obstruction caused by device obstruction, thereby significantly improving the safety of device after implantation in the body.

Specifically, the degradable material includes at least one of poly(DL-lactide-co-glycolide), poly(ethylene glycol)-poly(ε-caprolactone), poly(L-lactide-co-ε-caprolactone), poly(L-lactic acid), poly(L-lactic acid)-trimethyl chitosan, and poly(p-dioxanone).

Different degradable materials have different degradation times in an intestinal environment. Based on the required preset degradation time, a single degradable material or a blend of multiple degradable materials may be selected to form the degradable sleeve segment 110.

Generally, a high-incidence period of device obstruction after implantation of the sleeve 100 is within 15-180 days after implantation. Considering the decrease in mechanical performance as the degradable sleeve segment degrades, an expected degradation time of the degradable sleeve segment 110 may be set to 30-270 days.

Preferably, after the sleeve 100 is implanted into the digestive tract, an expected degradation time of the degradable sleeve segment 110 is 60-210 days.

Further, a plurality of degradable sleeve segments 110 are provided, and the plurality of degradable sleeve segments 110 are uniformly distributed along a length direction of the sleeve 100. When a plurality of degradable sleeve segments 110 are provided, the sleeve will break into a plurality of smaller sleeve segments after fracture, thereby further preventing the occurrence of intestinal obstruction.

Preferably, a preset degradation time of the plurality of degradable sleeve segments 110 decreases sequentially from the proximal end 101 to the distal end 102.

The sleeve segment close to the distal end 102 detaches first, while the sleeve segment close to the proximal end 101 detaches later. This design not only ensures effective prevention of intestinal obstruction but also allows the sleeve to remain in the digestive tract for as long as possible to continue delivering weight-loss benefits.

Further, other portions of the sleeve 100, excluding the degradable sleeve segments 110, are made of at least one material selected from PTFE, FEP, ePTFE, PU, LDPE, LLDPE, and PE.

Further, to ensure that the sleeve 100 has appropriate mechanical performance, the sleeve 100 has a wall thickness of 0.01-0.05 mm.

Further, the degradable sleeve segments 110 are connected to the other portions of the sleeve 100 by calendering (thermal pressing) or welding.

Specifically, the welding is laser welding or RF welding.

The digestive tract implant assembly provided by the present disclosure includes a stent 200 and the self-fracturing digestive tract sleeve 100 provided by the present disclosure, where the proximal end 101 of the self-fracturing digestive tract sleeve 100 is connected to the stent 200.

### Embodiment 1

The sleeve 100 provided by this embodiment has a length of 60cm. A degradable sleeve segment 110 is provided at a position 30cm from the proximal end 101. The degradable sleeve segment 110 has a length of 0.5 cm, and is made of a mixture of poly(L-lactide-co-ε-caprolactone) and poly(DL-lactide-co-glycolide) at a mass ratio of 8:2. A preset degradation time after implantation is 120 days.

Other portions of the sleeve 100, excluding the degradable sleeve segment 110, are made of PTFE. The sleeve 100 has a wall thickness of 0.016mm.

### Embodiment 2

The sleeve 100 provided by this embodiment has a length of 100cm. A degradable sleeve segment 110 is provided at a position 30cm from the proximal end 101. The degradable sleeve segment 110 has a length of 0.5cm and is made of poly(p-dioxanone) (PDO), and a preset degradation time after implantation is 180 days. Another degradable sleeve segment 110 is provided at a position 60cm from the proximal end. The degradable sleeve segment 110 has a length of 0.5 cm and is made of a mixture of poly(L-lactide-co-ε-caprolactone) and poly(DL-lactide-co-glycolide) at a mass ratio of 1:1, and a preset degradation time after implantation is 100 days.

Other portions of the sleeve 100, excluding the degradable sleeve segments 110, are made of LDPE/LLDPE. The sleeve 100 has a wall thickness of 0.02mm.

### Embodiment 3

The sleeve 100 provided by this embodiment has a length of 150cm. A degradable sleeve segment 110 is provided at a position 40cm from the proximal end 101. The degradable sleeve segment 110 has a length of 0.5 cm and is made of a mixture of poly(L-lactic acid)-trimethyl chitosan and poly(DL-lactide-co-glycolide) at a mass ratio of 2:8, and a preset degradation time after implantation is 30 days. Another degradable sleeve segment 110 is provided at a position 80cm from the proximal end 101. The another degradable sleeve segment 110 has a length of 0.5 cm and is made of a mixture of poly(L-lactide-co-ε-caprolactone) and poly(DL-lactide-co-glycolide) at a ratio of 8:2, and a preset degradation time after implantation is 120 days. A yet another degradable sleeve segment 110 is provided at a position 120cm from the proximal end 101. The yet another degradable sleeve segment 110 has a length of 0.5 cm and is made of poly(L-lactic acid), and a preset degradation time after implantation is 270 days.

Other portions of the sleeve 100, excluding the degradable sleeve segments 110, are made of ePTFE/FEP. The sleeve 100 has a wall thickness of 0.015 mm.

### Comparative Example

The comparative example is substantially the same as Embodiment 1, except that the sleeve 100 does not include any degradable sleeve segment 110.

### Experimental Example 1

The sleeves prepared in Embodiments 1-3 were completely immersed in simulated intestinal fluid (preparation method referring to Pharmacopoeia of the People's Republic of China, 2022 edition). The sleeves were oscillated on a shaker at 37±2 °C and 40 rpm for different durations until a preset time was reached. The sleeves were then carefully removed from the simulated intestinal fluid, and surface liquid was absorbed using absorbent paper. Both ends (excluding the degradable sleeve segments) of the sleeve (including the degradable sleeve segments) were fixed to the clamps of a computer-controlled materials testing machine. A test speed was set at 20 mm/min, and connection force before degradation was measured. When the connection force was less than 2N, a failure time was considered to be reached (allowing rapid breakage under intestinal peristaltic pressure in the event of obstruction). The immersion time at this point was recorded as the failure time.

**Table 1 Failure time (days) of the degradable sleeve segments of each embodiment**

| Group | Sleeve segment 1 | Sleeve segment 2 | Sleeve segment 3 |
|---|---|---|---|
| Embodiment 1 | 85 | / | / |
| Embodiment 2 | 130 | 70 | / |
| Embodiment 3 | 200 | 88 | 15 |

| | | | |
|---|---|---|---|
| Note: Sleeve segments 1-3 are arranged in order from the distal end to the proximal end. | | | |

As shown by the data in Table 1, the degradable sleeve segments 110 on the sleeve 100 provided by the embodiments of the present disclosure all achieved triggering of the failure times, and a design of time gradient differences was also achieved.

### Experimental Example 2

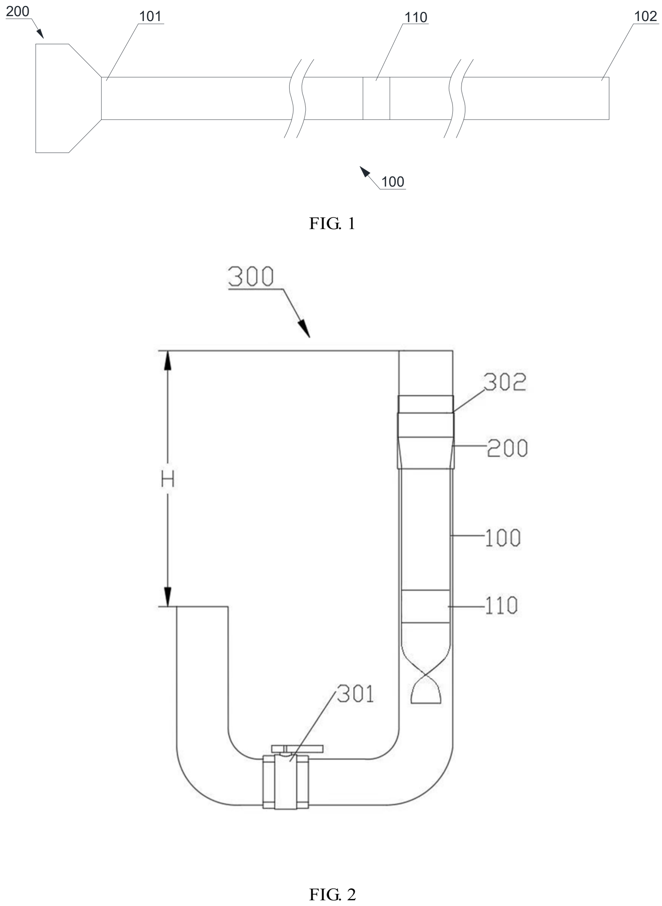

After obstruction of the sleeve 100, the sleeve 100 is subjected to pulling forces from peristalsis within the duodenum. According to a study by Gregerson et al., the maximum pressure generated by the intestinal peristalsis can reach 80 mmHg (108.8 cmH₂O) (Gregerson, H., et al. Essentials of Experimental Surgery: Gastroenterology. CRC Press, 1996). When the sleeve 100 becomes obstructed, even if the degradable sleeve segment 110 has not completely degraded, the partially degraded degradable sleeve segment 110 fractures under the pulling force of duodenal peristalsis, and the sleeve segment close to the distal end 102 is expelled from the body with intestinal peristalsis. At the same time, the degradable sleeve segments 110 provided can withstand normal intestinal peristaltic force without obstruction of the sleeve 100, and degrade within an expected time period. Based on this principle, a simulator for simulating intestinal obstruction generation is designed to replicate the detachment behavior of the self-fracturing digestive tract sleeve 100 provided by the present disclosure when the digestive tract sleeve 100 becomes obstructed, as shown in FIG. 2. The simulator 300 is capable of providing a pressure difference H and includes an adjustable valve 301 and a placement port 302 for receiving the digestive tract implant assembly provided by the present disclosure.

As shown in FIG. 2, the adjustable valve 301 was closed, and water was injected into the device until the water surface reached approximately 2cm from the placement port 302. The sleeve 100 was sealed with a thread to simulate obstruction. The digestive tract assembly, formed by the self-fracturing digestive tract sleeves 100 provided by Embodiment 1 and the comparative example, was placed in the placement port 302. Water was gradually injected into the sleeve 100 (it was ensured that the space outside the sleeve 100 was filled with water before water injection into the sleeve 100). Water was then injected in different amounts to adjust the pressure difference to 100 cmH₂O. Subsequently, the adjustable valve 301 was opened to record whether fracture occurs.

The above experiment was conducted after the sleeve 100 of Embodiment 1 had been implanted in the simulated intestinal environment for 100 days without fracturing, and the sleeve 100 was then placed in the simulator 300 to perform the simulation experiment. The experimental results were recorded in Table 2.

**Table 2 Fracture records of Embodiment 1 and the comparative example**

| Group | Fracture of the sleeve with the distal end not closed under 100 cmH₂O | Fracture of the sleeve with the distal end closed (simulated obstruction) under 100 cmH₂O |
|---|---|---|
| Embodiment 1 | No | Yes |
| Comparative Example | No | No |

As shown in Table 2, even if device obstruction occurs before degradation and fracture of the sleeve provided by the embodiment of the present disclosure, fracture can still be achieved under the pulling force of intestinal peristalsis.

In summary, the self-fracturing digestive tract sleeve provided by the embodiments of the present disclosure includes at least one degradable sleeve segment. This design ensures that after implantation of the sleeve in the digestive tract for a period of time, the degradable sleeve degrades and fractures in the digestive tract environment, thereby causing the sleeve segment from the degradation site to the distal end to detach. By presetting the degradation time to occur before potential device obstruction, the detachment of the sleeve segment closer to the distal end can effectively prevent intestinal obstruction caused by device obstruction. Alternatively, even if the degradable sleeve segment has not fully degraded before device obstruction occurs, the mechanical performance of the degradable sleeve segment is compromised due to partial degradation. Consequently, under intestinal peristaltic forces, it can still fracture and detach when device obstruction occurs. In this connection, the sleeve provided by the embodiment of the present disclosure effectively prevents intestinal obstruction caused by device obstruction, thereby significantly improving the safety of device after implantation in the body.

The above described contents are only preferred embodiments of the present disclosure and are not intended to limit the present disclosure. For those skilled in the art, the present disclosure can be modified and varied. Any modification, equivalent replacement, improvement, or the like made without departing from the spirit and principle of the present disclosure shall fall within the protection scope of the present disclosure.

### INDUSTRIAL APPLICABILITY

The graduated-release digestive tract implant sleeve provided by the present disclosure includes at least one degradable sleeve segment. This design ensures that after implantation of the sleeve in the digestive tract for a period of time, the degradable sleeve degrades and fractures in the digestive tract environment, thereby causing the sleeve segment from the degradation site to the distal end to detach. By presetting the degradation time to occur before potential device obstruction, the detachment of the sleeve segment closer to the distal end can effectively prevent intestinal obstruction caused by device obstruction. Alternatively, even if the degradable sleeve segment has not fully degraded before device obstruction occurs, the mechanical performance of the degradable sleeve segment is compromised due to partial degradation. Consequently, under intestinal peristaltic forces, it can still fracture and detach when device obstruction occurs. In this connection, the sleeve provided by the embodiment of the present disclosure effectively prevents intestinal obstruction caused by device obstruction, thereby significantly improving the safety of device after implantation in the body.

## Claims

1. A self-fracturing digestive tract sleeve, **characterized in that** the sleeve has a length of 60-150 cm and comprises a proximal end and a distal end, and at least one degradable sleeve segment made of a degradable material is provided on the sleeve at a position 30-120cm from the proximal end.

2. The self-fracturing digestive tract sleeve according to claim 1, wherein the degradable material comprises at least one of poly(DL-lactide-co-glycolide), poly(ethylene glycol)-poly(ε-caprolactone), poly(L-lactide-co-ε-caprolactone), poly(L-lactic acid), poly(L-lactic acid)-trimethyl chitosan, and poly(p-dioxanone).

3. The self-fracturing digestive tract sleeve according to claim 1 or 2, wherein an expected degradation time of the degradable sleeve segment is 30-270 days after the sleeve is implanted into the digestive tract.

4. The self-fracturing digestive tract sleeve according to claim 3, wherein a plurality of degradable sleeve segments are provided, and the plurality of degradable sleeve segments are uniformly distributed along a length direction of the sleeve.

5. The self-fracturing digestive tract sleeve according to claim 4, wherein a preset degradation time of the plurality of degradable sleeve segments decreases sequentially from the proximal end to the distal end.

6. The self-fracturing digestive tract sleeve according to claim 1, wherein other portions of the sleeve, excluding the degradable sleeve segment, are made of at least one material selected from PTFE, FEP, ePTFE, PU, LDPE, LLDPE, and PE.

7. The self-fracturing digestive tract sleeve according to claim 1, wherein the sleeve has a wall thickness of 0.01-0.05mm.

8. The self-fracturing digestive tract sleeve according to claim 1, wherein the degradable sleeve segment is connected to other portions of the sleeve by calendering or welding.

9. The self-fracturing digestive tract sleeve according to claim 8, wherein the welding is laser welding or RF welding.

10. A digestive tract implant assembly, **characterized by** comprising a stent and the self-fracturing digestive tract sleeve according to any one of claims 1 to 9, wherein the proximal end of the self-fracturing digestive tract sleeve is connected to the stent.
